# FASCICULE DE BREVET EUROPEEN

(11) **EP 3 687 383 B1**
(45) Date de publication et mention de la délivrance du brevet: **16.10.2024**
(21) Numéro de dépôt: 18786837.7
(22) Date de dépôt: 21.09.2018
(51) Int. Cl.: A61B 5/00

(54) **ELECTROGUSTOMETRE**
ELEKTROGUSTOMETER
ELECTROGUSTOMETER

(30) Priorité: 25.09.2017 FR 1758832
(43) Date de publication de la demande: 05.08.2020
(73) Titulaire: MY ROBOTICS, 94160 Saint-Mandé (FR)
(72) Inventeur: BASTIAN, Pierre-Antoine, 75005 Paris (FR)
(74) Mandataire: Cabinet Netter
(86) Numéro de dépôt international: PCT/FR2018/052303
(87) Numéro de publication internationale: WO 2019/058063

(56) Documents cités:
- FR-A1- 2 626 761
- US-A1- 2008 234 604
- LOUDON MALCOLM ET AL: "A computer-controlled electrogustometer for the estimation of evoked taste thresholds", BEHAVIOR RESEARCH METHODS, INSTRUMENTS AND COMPUTERS, vol. 29, no. 3, 1 September 1997 (1997-09-01), US, pages 358 - 363, XP055966955, ISSN: 0743-3808, Retrieved from the Internet <URL:https://link.springer.com/content/pdf/10.3758/BF03200588.pdf> [retrieved on 20230821], DOI: 10.3758/BF03200588
- ANIRBAN BANERJEE: "Development of an automated electrogustometer", 1 January 2011, SUSSEX RESEARCH ONLINE, pages: 1 - 200, XP055479179
- FITZSIMONS M ET AL: "The application of d.c. electrical stimulation in evoking and recording gustatory brain potentials", PHYSIOLOGICAL MEASUREMENT, INSTITUTE OF PHYSICS PUBLISHING, BRISTOL, GB, vol. 20, no. 4, 1 November 1999 (1999-11-01), pages 385 - 400, XP020074005, ISSN: 0967-3334, DOI: 10.1088/0967-3334/20/4/306
- H TOMITA H ET AL: "Clinical use of electrogustometry: strengths and limitations.", ACTA OTOLARYNGOL SUPPL., 1 January 2002 (2002-01-01), pages 27 - 38, XP055478808, Retrieved from the Internet <URL:http://www.ncbi.nlm.nih.gov/pubmed/12132619> [retrieved on 20180528]

## Description

L'invention, telle que définie par la revendication indépendante 1, concerne le domaine de la gustométrie, et en particulier de l'électrogustométrie.

Le goût est un sens complexe dont les mécanismes sont assez peu connus. Néanmoins, le trouble du goût, lorsque quantifié, peut être très utile dans le suivi médical, par exemple dans le suivi des patients en oncologie, la prévention et l'aide au sevrage en tabacologie et le diagnostic précoce de certaines maladies, par exemple la maladie d'Alzheimer et Parkinson.

La gustométrie vise à analyser les troubles du goût, par l'analyse de la perception du goût par une personne en éliminant un trouble de l'odorat. Trois familles de procédures sont à la disposition des médecins : la gustométrie chimique, l'électrogustométrie et la biopsie des papilles pour analyser le tissu des bourgeons gustatifs.

La gustométrie chimique consiste à déposer sur la langue d'un patient un ou des produits chimiques et à mesurer sa ou ses réactions. L'électrogustométrie consiste à mesurer la détection du goût par un patient grâce à l'application d'un très faible courant sur sa langue, le but étant de mesurer le courant le plus faible possible qui va provoquer l'apparition du goût qu'une personne est capable de ressentir.

La gustométrie chimique est peu pratique pour de nombreuses raisons : temps de réaction du patient, temps de relaxation du patient, difficulté de doser, d'appliquer des procédures répétables, etc. A cela s'ajoute la nécessité d'avoir un laboratoire de chimie, le long temps de préparation, les conditions de stockage des produits chimiques contraignantes, etc.

L'électrogustométrie, par son utilisation de l'électricité semblerait de prime abord avoir plus de potentiel. Pourtant, à ce jour, la Demanderesse ne connaît qu'un seul et unique appareil d'électrogustométrie qui semble avoir fait l'objet d'une production commerciale, le TR-06 Rion fabriqué par la société Sensonics, INC.

Il s'agit d'un appareil assez rudimentaire, dans lequel le courant appliqué est délivré entre 4 µA et 400 µA avec un pas de discrétisation moyen de 20µA.

L'application d'un stimulus est uniquement manuelle, contrôlée depuis un pédalier pour activer l'envoi du courant, et l'opérateur note sur papier les paramètres de la stimulation appliquée au patient avant de faire les calculs de seuil électrogustométrique par lui-même. Un certain temps de récupération est donné au patient pour que le goût acide d'une stimulation disparaisse, mais ce temps dépend de l'utilisateur. De même, le temps de stimulation n'est programmable au maximum qu'à 2 secondes de durée de stimulation, alors que le temps de réaction d'un patient est très largement variable. Le TR-06 Rion est encombrant, et présente un poids total de 2,3 kg, ce qui en fait un appareil lourd, volumineux et difficilement transportable. De par la complexité de son fonctionnement et le manque d'automatisation du TR-06 Rion, son utilisation est aujourd'hui purement clinique et exploratoire, sans qu'il soit envisageable de l'utiliser quotidiennement par un médecin avec ses patients.

Son échelle le rend très peu précis, son encombrement, sa prise en main complexe et ses limitations technologiques le rendent très peu pratique, au point que ce dispositif ne semble pas être réellement utilisé dans la pratique, y compris pour les études cliniques. A ce sujet, l'abrégé de l'article « A comparison of two electric taste stimulation devices » de McClure ST et al, Physiol Behav. 2007 Nov 23;92(4):658-64, dit, en comparant le TR-06 Rion à un électrogustomètre improvisé avec une pile de 1,6V conclut de la manière suivante : « Le dispositif à pile peut fournir une alternative portable peu chère à un électrogustomètre pour l'utilisation dans l'étude clinique du goût ».

Pour ces raisons, l'électrogustométrie est à ce jour extrêmement peu développée, et même remplacée par la gustométrie chimique malgré les désavantages décrits plus haut. Il existe donc un réel besoin d'offrir un électrogustomètre médical, qui puisse être utilisé par des médecins quotidiennement avec leurs patients, dans le cadre de protocoles permettant un suivi de la perception du goût des patients, aussi bien dans le cadre d'adaptation de posologie, de prévention, d'aide au sevrage ou de diagnostic d'une maladie.

Il est à noter que l'électrogustométrie a fait l'objet de plusieurs publications, notamment celle d'Anirban Banerjee « Development of an automated electrogustometer » (Sussex Research Online, XP055479179, pages 1-200, 1er janvier 2011) et celle de Loudon Malcolm et al. « A computer-controlled electrogustometer for the estimation of evoked taste threshold » (Behavior Research Methods, Instruments & Computers, vol. 29, n°3, XP055966955, pages 358-363, 1er septembre 1997).

A cet effet, l'invention propose un électrogustomètre selon la revendication indépendante 1.

Cet électrogustomètre est avantageux car il permet de réaliser des protocoles de mesure de gustométrie fiables. De plus, de par sa précision, il peut être utilisé dans un milieu médical au jour le jour. En effet, ses caractéristiques permettent à cet électrogustomètre d'être très précis (à 1% près par rapport à ce que l'utilisateur souhaite envoyer comme courant), modulable selon les besoins de l'utilisateur et de respecter les contraintes réglementaires qui lui permettent d'être certifié Dispositif Médical de type IIa. Ainsi, il n'y a pas de risque d'envoyer trop de courant dans la langue du patient, ni de se tromper dans les calculs de mesure de seuil électrogustométrique, tout en permettant de réaliser un test de sensibilité au goût en à peine quelques minutes.

Dans diverses variantes, l'électrogustomètre selon l'invention peut présenter une ou plusieurs des caractéristiques suivantes :
- le générateur de courant comprend un montage à transistor bipolaire.
- l'électrogustomètre -comprend en outre une électrode pour appliquer un courant de stimulation généré par le générateur de courant,
- l'interface comprend un dispositif électronique déporté relié à un microcontrôleur,
- le dispositif électronique déporté comprend un processus de détermination automatique d'un seuil électrogustométrique basé sur un ou plusieurs retours en réponse suite à l'application d'une série de courant de stimulation générés par le générateur de courant,
- le dispositif électronique comprend un processus de détermination automatique d'un seuil électrogustométrique basé sur l'algorithme de Dixon,
- le dispositif électronique est agencé pour utiliser un processus en double aveugle,
- le dispositif électronique déporté est agencé pour utiliser un processus dans lequel l'application d'une série de courant de stimulation générés par le générateur de courant comprend l'émission de courants de stimulation pendant une durée fixe, à partir d'une valeur d'intensité de départ, chaque courant de stimulation étant séparé de l'émission qui le précède par une valeur d'intensité fixe,
- le dispositif électronique comprend un processus de détermination automatique d'un seuil électrogustométrique basé sur une progression dichotomique, et
- le convertisseur numérique analogique reçoit une commande numérique sous forme d'un nombre, et en ce que chaque valeur de tension discrète correspond à une commande numérique.

D'autres caractéristiques et avantages de l'invention apparaîtront mieux à la lecture de la description qui suit, tirée d'exemples donnés à titre illustratif et non limitatif, tirés des dessins sur lesquels :
- la figure 1 représente une vue schématique d'un électrogustomètre médical selon l'invention,
- la figure 2 représente un schéma électrique de principe d'un élément de la figure 1,
- la figure 3 représente un exemple de mise en oeuvre d'une fonction par un élément de la figure 1, et
- la figure 4 représente un exemple de mise en oeuvre d'une fonction en variante par un élément de la figure 1.

Les dessins et la description ci-après contiennent, pour l'essentiel, des éléments de caractère certain. Ils pourront donc non seulement servir à mieux faire comprendre la présente invention, mais aussi contribuer à sa définition, le cas échéant.

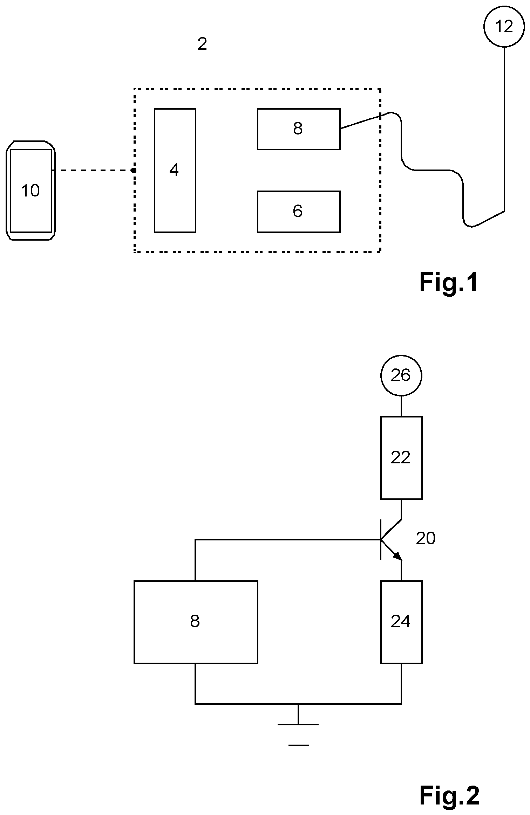

La figure 1 représente un diagramme schématique d'un électrogustomètre 2 selon l'invention.

L'électrogustomètre 2 comprend un microcontrôleur 4, un convertisseur numérique-analogique 6 et un générateur de courant 8.

Le microcontrôleur 4 reçoit des consignes d'intensité de stimulation d'un téléphone 10, et le générateur de courant 8 émet en réponse un courant à une électrode 12. Ainsi, avec le téléphone 10, l'électrogustomètre 2 et l'électrode 12, il est possible de conduire une procédure d'électrogustométrie en disposant l'électrode 12 sur la langue d'un patient et en recueillant les réponses de ce dernier à des stimulations.

Dans l'exemple décrit ici, le microcontrôleur 4 est du type ATMega168 de la marque Microchip. Le microcontrôleur 4 est agencé pour recevoir les consignes de stimulation du téléphone 10 et pour les convertir en commande numérique. Le microcontrôleur 4 joue donc le rôle d'une interface permettant de commander l'intensité du courant émise par l'électrogustomètre 2. Le téléphone 10 est dans l'exemple décrit ici du type smartphone et communique par un protocole sans fil avec le microcontrôleur 4 comme le Bluetooth. D'autres protocoles sans fil pourraient être utilisés, et le téléphone 10 pourrait être relié par un câble (par exemple USB) à une entrée du microcontrôleur 4. Le téléphone 10 pourrait être remplacé par une tablette ou par tout autre dispositif électronique déporté adapté à exécuter un algorithme comme décrit plus bas et permettant de communiquer avec l'interface de l'électrogustomètre 2. Toujours en variante, le téléphone 10 pourrait être intégré à l'électrogustomètre 2 afin d'offrir une interface directement accessible. La conversion de la consigne de stimulation en commande numérique peut être basée sur une règle de correspondance, sur une table de recherche de type look-up table, ou sur une commande directe avec une boucle de rétroaction.

La figure 2 représente un exemple de schéma électrique mis en oeuvre par le générateur de courant 8 pour transformer la tension émise en sortie du convertisseur numérique-analogique 6 en courant pour l'électrode 12.

Comme on peut le voir sur cette figure, la sortie du convertisseur numérique analogique 8 est reliée à la base d'un transistor bipolaire 20. L'émetteur du transistor 20 est relié en série avec une résistance 22 qui représente la résistance de la langue du patient lorsque celle-ci est reliée à l'électrode 12, tandis que le collecteur du transistor 20 est relié en série à une résistance 24 qui est à la masse. La résistance 22 est également en série avec l'autre partie de l'électrode 12 qui est en série avec une source de tension continue 26. Pour fermer le circuit lorsqu'un courant de stimulation est émis, la personne qui reçoit l'électrode 12 porte un bracelet (non représenté sur la figure 2) qui est relié à la masse en aval de la résistance 24.

Selon ce schéma, l'intensité qui parcourt la résistance 22 est l'intensité ic au niveau du collecteur du transistor 20. Dès lors que la tension entre le collecteur et l'émetteur du transistor 20 reste positive, l'intensité ic est sensiblement égale à l'intensité ie au niveau de l'émetteur.

Ainsi, l'intensité ic est égale à la différence entre la tension de sortie du convertisseur numérique analogique 6 et la tension entre le collecteur et la base du transistor 20, divisée par la résistance 24.

Dans l'exemple décrit ici, le convertisseur numérique-analogique 6 est du type MCP4725 de la société Microchip. L'emploi d'un convertisseur analogique-numérique est particulièrement avantageux car il permet de contrôler avec précision la tension qui sort de ce dernier. Ainsi, en combinaison avec le générateur de courant 8, il est possible de maîtriser l'intensité du courant émis à l'électrode 12 à 0,1 µA près.

En variante, le générateur de courant 8 pourrait également être basé sur un circuit à transistor à effet de champ, ou sur un montage à amplificateur opérationnel moyennant un refroidissement adapté.

La Demanderesse a découvert que l'électrogustomètre 2 décrit avec les figures 1 et 2 est particulièrement avantageux car il permet de mettre en oeuvre des algorithmes permettant de diagnostiquer rapidement, automatiquement et avec précision le seuil électrogustométrique d'un patient.

Ainsi, la Demanderesse a découvert que l'algorithme de Dixon décrit dans le livre Dixon & Massey « *Introduction to Statistical Analysis »,* Mac Graw Hill en 1960. Historiquement, l'algorithme de Dixon a été développé pour déterminer avec précision la quantité d'explosif pour faire exploser une quantité choisie de roche. Bien qu'il n'existe aucun lien direct avec l'électrogustométrie, la Demanderesse a découvert que l'application de l'algorithme de Dixon avec l'électrogustomètre de l'invention donne des résultats extrêmement précis, extrêmement rapidement et de manière sûre.

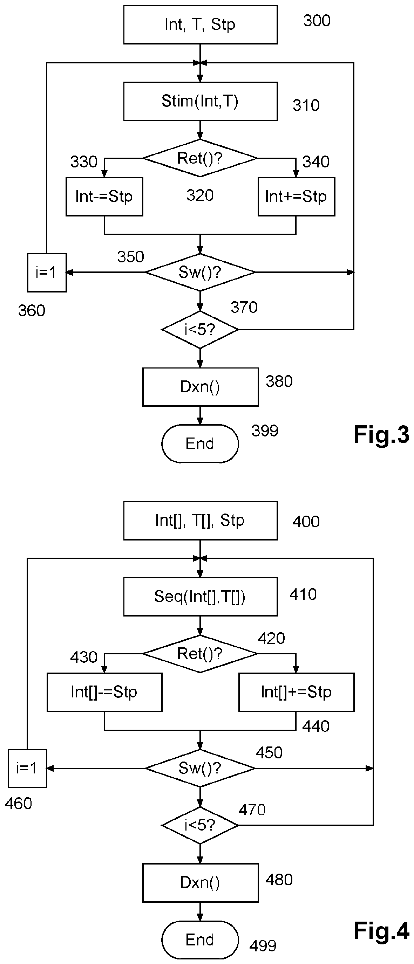

La figure 3 représente un exemple de mise en oeuvre d'une première version de l'algorithme de Dixon avec l'électrogustomètre 2. Dans une opération 300, l'intensité de départ Int est fixée, ainsi que le pas Stp et la durée de stimulation T. Ces paramètres peuvent par exemple être introduits via une application exécutée par le téléphone 10 et qui va commander l'électrogustomètre 2. Le téléphone 10 peut être tenu par l'utilisateur, qui demandera ensuite au patient s'il a ressenti ou non la stimulation. Cela permet d'éviter d'influencer psychologiquement le patient et ne pas l'inciter à mentir sur son ressenti.

Une fois les paramètres fixés, une boucle de stimulations est exécutée. Dans une opération 310, le téléphone 10 exécute une fonction Stim() qui commande à l'électrogustomètre 2 d'émettre un courant continu d'intensité Int pour une durée T à l'électrode 12.

En réponse à la stimulation, le patient indique dans une opération 320 s'il a ressenti la stimulation ou pas. Si le patient indique avoir ressenti une stimulation, alors l'intensité Int est décrémentée avec le pas Stp dans une opération 330. Si le patient indique ne pas avoir ressenti une stimulation, alors l'intensité est incrémentée avec le pas Stp dans une opération 340.

Une fonction Sw() est ensuite exécutée dans une opération 350. Cette fonction compare le résultat de la boucle courante avec celui de la boucle précédente. Si la réponse du patient est la même, alors la boucle reprend avec l'opération 310. Sinon, un virage a été effectué, et 4 mesures complémentaires vont être réalisées pour caractériser la mesure.

Ainsi, dans une opération 360, un indice i est initialisé à la valeur 1. Ensuite, dans toutes les exécutions de la boucle, et jusqu'à la fin de la fonction, la fonction Sw() ne fera plus de test et se contentera d'incrémenter l'indice i. Cela peut par exemple être fait en activant un drapeau à la détection du virage par la fonction Sw(), le drapeau activé provoquant le contournement du test dans la fonction Sw() et l'incrémentation de l'indice i. D'autres variantes sont bien évidemment possibles.

Enfin, un test est réalisé dans une opération 370 afin de déterminer si les 4 opérations suivant la détection du virage ont été effectuées ou pas. Si c'est le cas, alors les réponses de l'utilisateur données sur les 5 dernières mesures sont utilisées conjointement avec la matrice de Dixon pour calculer le seuil de goût du patient grâce à une fonction Dxn() dans une opération 380, et la fonction se termine dans une opération 399. Sinon, la boucle reprend avec l'opération 310 pour les dernières mesures à réaliser.

La figure 4 représente une fonction en variante de la figure 3 dans laquelle l'algorithme de Dixon est toujours mis en oeuvre, mais sur le fondement cette fois d'un test en double aveugle. Pour cela, la stimulation et la mesure sont légèrement différentes en ce que deux stimulations vont être proposées à chaque fois au patient, dont une seule sera réelle. La réponse qui sera demandée au patient concerne celle des deux stimulations qui a effectivement eu lieu.

Ainsi, les opérations de la fonction de la figure 4 sont très proches de celles de la fonction de la figure 3. Elles diffèrent en ce que :
- il ne s'agit plus d'une intensité Int mais d'un vecteur Int[] qui reçoit l'intensité de la première et de la deuxième stimulation, ainsi que pour la durée T qui est un vecteur T[].

Les valeurs de Int[] sont uniquement Int et 0, et la durée est la même pour les deux stimulations,
- l'opération 410 n'est plus une stimulation directe, mais l'exécution d'une séquence par une fonction Seq() qui réalise la fonction de double-aveugle, c'est-à-dire que seul l'application dans le téléphone 10 sait laquelle de la première ou de la deuxième stimulation a été effectuée avec l'intensité Int pour une exécution de boucle donnée, et
- l'opération 420 n'est pas basée sur la réponse du patient uniquement, mais sur la comparaison entre sa réponse et la réalité qui est connue de l'application. Ainsi, si le patient a identifié correctement la stimulation qui a été faite, alors l'intensité est décrémentée, tandis que dans le cas inverse elle est augmentée.

D'autres algorithmes de test pourraient être mis en oeuvre par le biais d'une application sur le téléphone 10, comme un algorithme basé sur la dichotomie, ou autre. L'application peut aussi permettre à un utilisateur de commander directement l'électrogustomètre 2, sans passer par un algorithme. Dans ce cas-là, l'intensité et la durée sont directement spécifiées sur le téléphone 10 et, comme dans tous les protocoles, il est ensuite demandé par l'utilisateur au patient s'il a ressenti la stimulation ou pas. En variante, le patient peut utiliser l'électrogustomètre et l'application seul, avec un protocole de mesure simplifié. Dans ce cas, un bouton de type « STOP » apparaît sur l'écran de l'application mobile, que le patient tient d'une main, et, de l'autre main, il tient l'électrode positionnée sur sa langue. Tant que le patient n'appuie pas sur le bouton « STOP », le courant augmente peu à peu sur l'électrogustomètre et envoie une stimulation ayant toujours la même durée et le même pas d'incrémentation. Une fois que le patient appuie sur « STOP », le résultat s'affiche sur l'application. Si le courant atteint l'intensité maximum, le test est arrêté, et un message d'alerte est affiché sur le smartphone et l'utilisateur peut se voir proposer de recommencer.

Optionnellement, pour chaque protocole, si l'électrode est déconnectée de la langue du patient, alors un message d'erreur est affiché, la stimulation s'arrête et l'utilisateur peut reprendre le test avec la même valeur que précédemment.

L'électrogustomètre 2 décrit ici est d'autant plus avantageux qu'il est naturellement intégrable à un environnement connecté. En effet, comme il n'est plus commandable uniquement manuellement selon un protocole mis en oeuvre par un chercheur, mais par une interface électronique moderne, il peut naturellement s'intégrer dans un environnement connecté, et permettre d'intégrer aisément les résultats des mesures d'un patient à l'ensemble de ses données médicales. Cela permettra de créer la première base de données sur les troubles du goût, et à terme de mettre en relation les troubles du goût et autres données de santé provenant de carnets de santé connectés et autres dispositifs médicaux, et de mettre en relation trouble du goût et traitements médicaux avec analyse des effets secondaires d'un traitement, etc. l'invention est définie par les revendications qui suivent.

## Revendications

1. Electrogustomètre comprenant
une interface capable de recevoir une consigne de stimulation, et
un générateur de courant (8) agencé pour produire un courant de stimulation,
dans lequel l'interface est agencée pour tirer une commande numérique de la consigne de stimulation,
ledit électrogustomètre comprenant en outre un convertisseur numérique analogique (6) adapté pour convertir la commande numérique en une tension continue, les différentes commandes numériques induisant des valeurs de tension respectives discrètes,
dans ledit électrogustomètre le générateur de courant (8) est agencé pour convertir la tension continue en un courant de stimulation dont l'intensité est comprise entre 0 et 250 µA en fonction de la valeur de tension discrète et varie par pas fixe compris entre 0,05 µA et 1 µA,
l'interface comprend un dispositif électronique (10) relié à un microcontrôleur (4),
et le dispositif électronique (10) comprend un processus de détermination automatique d'un seuil électrogustométrique basé sur un ou plusieurs retours en réponse suite à l'application d'une série de courant de stimulation générés par le générateur de courant (8) selon l'algorithme de Dixon.

2. Electrogustomètre selon la revendication 1, dans lequel le générateur de courant (8) comprend un montage à transistor bipolaire.

3. Electrogustomètre selon la revendication 1 ou 2, comprenant en outre une électrode (12) pour appliquer un courant de stimulation généré par le générateur de courant (8).

4. Electrogustomètre selon l'une des revendications précédentes , dans lequel le dispositif électronique (10) est agencé pour utiliser un processus en double aveugle.

5. Electrogustomètre selon l'une des revendications précédentes, dans lequel le dispositif électronique (10) est agencé pour utiliser un processus dans lequel l'application d'une série de courant de stimulation générés par le générateur de courant (8) comprend l'émission de courants de stimulation pendant une durée fixe, à partir d'une valeur d'intensité de départ, chaque courant de stimulation étant séparé de l'émission qui le précède par une valeur d'intensité fixe.

6. Electrogustomètre selon l'une des revendications précédentes, dans lequel le convertisseur numérique analogique (6) reçoit une commande numérique sous forme d'un nombre, et en ce que chaque valeur de tension discrète correspond à une commande numérique unique.

## Patentansprüche

1. Elektrogustometer, umfassend
eine Schnittstelle, die in der Lage ist, eine Stimulationsanweisung zu empfangen, und
einen Stromgenerator (8), der dazu angeordnet ist, einen Stimulationsstrom zu erzeugen,
wobei die Schnittstelle dazu angeordnet ist, aus der Stimulationsanweisung einen digitalen Befehl zu gewinnen,
wobei das Elektrogustometer ferner einen Digital-Analog-Wandler (6) umfasst, der dazu ausgelegt ist, den digitalen Befehl in eine Gleichspannung umzuwandeln, wobei die verschiedenen digitalen Befehle diskrete jeweilige Spannungswerte induzieren,
im Elektrogustometer ist der Stromgenerator (8) dazu angeordnet, die Gleichspannung in einen Stimulationsstrom umzuwandeln, dessen Intensität zwischen 0 und 250 µA in Abhängigkeit vom diskreten Spannungswert beträgt und in festen Schritten zwischen 0,05 und 1 variiert,
wobei die Schnittstelle eine elektronische Vorrichtung (10) umfasst, die mit einem Mikrocontroller (4) verbunden ist,
und die elektronische Vorrichtung (10) ein Verfahren zum automatischen Bestimmen eines elektrogustometrischen Schwellenwerts auf der Grundlage einer oder mehrerer Rückmeldungen nach dem Anwenden einer Reihe von Stimulationsströmen umfasst, die vom Stromgenerator (8) gemäß dem Dixon-Algorithmus erzeugt werden.

2. Elektrogustometer nach Anspruch 1, wobei der Stromgenerator (8) eine bipolare Transistoranordnung umfasst.

3. Elektrogustometer nach Anspruch 1 oder 2, das ferner eine Elektrode (12) zum Anwenden eines vom Stromgenerator (8) erzeugten Stimulationsstroms umfasst.

4. Elektrogustometer nach einem der vorhergehenden Ansprüche, wobei die elektronische Vorrichtung (10) dazu angeordnet ist, ein Doppelblindverfahren zu verwenden.

5. Elektrogustometer nach einem der vorhergehenden Ansprüche, wobei die elektronische Vorrichtung (10) dazu angeordnet ist, ein Verfahren, bei dem das Anwenden einer Reihe von Stimulationsströmen, die vom Stromgenerator (8) erzeugt werden, das Ausgeben von Stimulationsströmen während eines festen Zeitraums umfasst, anhand eines anfänglichen Intensitätswerts zu verwenden, wobei jeder Stimulationsstrom durch einen festen Intensitätswert von der vorhergehenden Emission getrennt ist.

6. Elektrogustometer nach einem der vorhergehenden Ansprüche, wobei der Digital-Analog-Wandler (6) einen digitalen Befehl in Form einer Zahl empfängt, und dass jeder diskrete Spannungswert einem eindeutigen digitalen Befehl entspricht.

## Claims

1. Electrogustometer comprising
an interface capable of receiving a stimulation instruction, and
a current generator (8) arranged to produce a stimulation current,
in which the interface is arranged to obtain a digital command from the stimulation instruction,
said electrogustometer further comprising a digital-to-analog converter (6) arranged to convert the digital command into a DC voltage, the different digital commands inducing discrete respective voltage values,
in said electrogustometer the current generator (8) is arranged to convert the DC voltage into a stimulation current with an intensity of between 0 and 250 depending on the discrete voltage value and varies by fixed steps of between 0.05 and 1 µA,
the interface comprises an electronic device (10) connected to a microcontroller (4),
and the electronic device (10) comprises a process for automatically determining an electrogustometric threshold based on one or more feedbacks in response to the application of a series of stimulation currents generated by the current generator (8) based on Dixon's algorithm.

2. Electrogustometer according to claim 1, wherein the current generator (8) comprises a bipolar transistor assembly.

3. Electrogustometer according to claim 1 or 2, further comprising an electrode (12) for applying a stimulation current generated by the current generator (8).

4. Electrogustometer according to one of the preceding claims, wherein the electronic device (10) is arranged to use a double-blind process.

5. Electrogustometer according to one of the preceding claims, wherein the electronic device (10) is arranged to use a process in which the application of a series of stimulation currents generated by the current generator (8) comprises the emission of stimulation currents for a fixed time, from an initial intensity value, each stimulation current being separated from the preceding emission by a fixed intensity value.

6. Electrogustometer according to one of the preceding claims, wherein the digital-to-analog converter (6) receives a digital command in the form of a number, and in that each discrete voltage value corresponds to a unique digital command.
